# FASCICULE DE BREVET EUROPEEN

(11) **EP 0 354 944 B1**
(45) Date de publication et mention de la délivrance du brevet: **23.06.1993**
(21) Numéro de dépôt: 89901565.5
(22) Date de dépôt: 19.01.1989
(51) Int. Cl.: G01N 33/08

(54) **OVOSCOPE TRIEUR AUTOMATIQUE**
AUTOMATISCHES EIERPRÜFGERÄT
AUTOMATIC SORTER OVOSCOPE

(30) Priorité: 20.01.1988 FR 8800628
(43) Date de publication de la demande: 21.02.1990
(73) Titulaire: BREUIL, S.A., F-29230 Landivisiau (FR)
(72) Inventeur: KEROMNES, Bernard, F-29210 Morlaix (FR); BREUIL, Jean-Pierre, F-29231 Taule (FR)
(74) Mandataire: Chambon, Gérard
(86) Numéro de dépôt international: FR8900015
(87) Numéro de publication internationale: WO8906797

(56) Documents cités:
- DE-C- 1 287 741
- FR-A- 2 455 282
- GB-A- 968 962
- US-A- 1 987 336

## Description

L'invention concerne un ovoscope trieur automatique.

Il est connu de mirer les oeufs pour en observer l'intérieur afin de s'assurer de l'état de leur contenu.

Cette opération de mirage a notamment pour but de vérifier l'état d'incubation ou de rechercher les diverses altérations éventuelles des oeufs.

Il est notamment courant de procéder au mirage des oeufs vers le 18ème jour d'incubation afin de pouvoir transférer vers des paniers d'éclosion les "bons oeufs" et éliminer les oeufs clairs, c'est-à-dire les oeufs non fécondés. Il est possible aussi de procéder à cette opération de mirage en vue de retirer les oeufs couvis, les oeufs envahis par des moisissures, les oeufs putréfiés, ceux contenant des embryons morts, etc. Le mirage se faisait jusqu'à présent directement à la main ou au moyen d'un mire-oeufs ou ovoscope. L'ovoscope classique est équipé d'une source lumineuse pour permettre d'apprécier à l'oeil l'état de transparence des oeufs. On a toutefois cherché à concevoir des appareils plus sophistiqués susceptibles de mesurer l'état de transparence des oeufs.

Un autre problème à l'automatisation de ce type d'opération réside dans le triage après mirage entre les bons oeufs et les oeufs défectueux.

L'invention propose un ovoscope trieur automatique, qui est remarquable en ce qu'il comporte une boîte à lumière aménagée audessous d'une tête de mirage qui est montée de manière mobile verticalement au-dessus des oeufs à mirer et d'un convoyeur d'évacuation, et qui est munie d'au moins une ventouse destinée à saisir par aspiration des oeufs à l'unité et équipée d'un moyen de mise sous vide et d'un moyen sensible à la lumière, tandis qu'un dispositif de commande du moyen de mise sous vide permet d'appliquer par aspiration chaque ventouse sur un oeuf à mirer et d'en apprécier la transparence en fonction de l'importance de la lumière provenant de la boîte à lumière et parvenant au moyen sensible, tandis que le dispositif de commande est asservi au signal dudit moyen sensible à la lumière, de manière telle que ladite ventouse puisse retenir par aspiration l'oeuf lorsque le signal du moyen sensible à la lumière indique le dépassement d'une certaine transparence prédéterminée dudit oeuf et permettre de le déplacer verticalement en même temps que la tête de mirage pour le relâcher sur ledit convoyeur d'évacuation.

Avantageusement, la tête de mirage est montée de manière mobile verticalement également au-dessus d'un convoyeur d'acheminement des oeufs à mirer, le tout de manière, après mirage, à évacuer les oeufs défectueux au moyen du convoyeur d'évacuation, à récupérer les bons oeufs au moyen du convoyeur d'acheminement et à amener un nouveau lot d'oeufs à mirer au moyen dudit convoyeur d'acheminement.

Selon un mode de réalisation, il comporte en outre un convoyeur de transfert destiné à recevoir, après mirage, les bons oeufs, qui sont transférés au moyen de la tête de mirage, du convoyeur d'acheminement, audit convoyeur de transfert.

De préférence, le moyen de mise sous vide de chaque ventouse comporte un conduit d'aspiration dont l'une des extrémités débouche dans le fond de ladite ventouse et dont l'autre extrémité est reliée à une pompe à vide par l'intermédiaire d'une électrovanne, tandis que le moyen sensible à la lumière est un phototransistor à infrarouge, et que la boîte à lumière est aménagée pour produire de la lumière infrarouge.

Pour contrôler le bon fonctionnement de l'appareil, il peut comporter un système de contrôle qui permet de contrôler la détection correcte d'absence de lumière par chaque moyen sensible lorsque la boîte à lumière est éteinte et que la ventouse en cause est appliquée sous vide sur un oeuf. Dans ce cas, il est de préférence pourvu d'un moyen de visualisation du mauvais fonctionnement de la ventouse contrôlée, ainsi que d'un moyen qui permet d'isoler ladite ventouse en cas de défaillance détectée.

Selon un mode de réalisation, il comporte par ventouse, un comparateur aménagé pour comparer le signal du moyen correspondant sensible à la lumière avec au moins un signal prédéterminé, généré par un générateur de signaux de seuil, de manière à créer un signal de détection qui est fonction de la comparaison desdits signaux. Alors, le générateur de signaux de seuil est aménagé pour générer au moins un signal prédéterminé de comparaison qui correspond à un seuil choisi de transparence, de manière à pouvoir créer un signal de détection qui est fonction du dépassement ou non de ce seuil. De préférence, le générateur de signaux de seuil est aménagé pour générer en outre un signal prédéterminé de comparaison qui correspond à un seuil d'absence de lumière, de manière à pouvoir créer un signal de détection, dit de "test noir", qui est fonction du dépassement ou non de ce seuil.

Dans le cas d'un mode de réalisation du type précité, le signal de détection, après mémorisation, peut servir pour agir sur la commande du moyen de mise sous vide, de manière à maintenir la mise sous vide de la ventouse correspondante lorsque ledit signal indique un dépassement du seuil choisi.

De préférence, encore, il comporte un automate de contrôle, qui permet de commander le choix du signal prédéterminé de comparaison, de commander la mise en mémoire du signal de détection et de commander un éventuel arrêt de l'appareil lorsque le signal de test noir branché en retour sur ledit automate, est mauvais.

En outre, il peut être intéressant de connaître les proportions entre les bons oeufs et les oeufs défectueux selon les arrivages. Un appareil selon l'invention permet justement d'établir facilement ces ratios. Pour cela, il suffit, par exemple, qu'il comporte un moyen de mesure du courant électrique utilisé par une partie au moins de l'appareil et un calculateur aménagé de manière à déterminer en fonction des informations dudit appareil de mesure, des rapports numériques entre le nombre d'oeufs défectueux et le nombre des bons oeufs.

L'invention sera bien comprise et d'autres particularités apparaîtront à la lecture de la description qui va suivre et qui se réfère aux dessins annexés dans lesquels:
- la figure 1 montre en élévation un appareil selon l'invention,
- la figure 2 montre en détail l'une des ventouses de mesure et de préhension,
- la figure 3 est un schéma général de commande de l'appareil,
- les figures 4 et 5 représentent plus en détail les cartes électroniques de la figure 3.

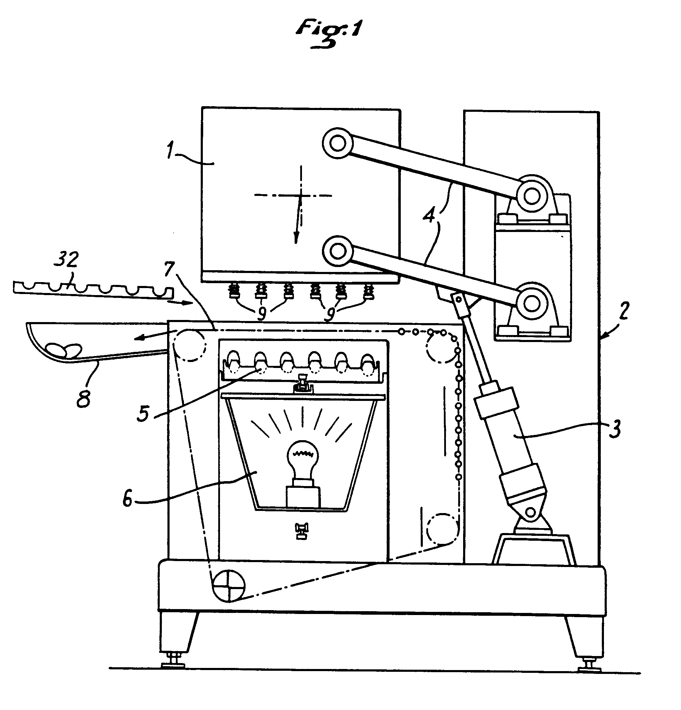

La figure 1 montre une tête 1, dite de mirage, qui est montée de manière mobile verticalement sur un chassis 2 au moyen d'au moins un vérin 3 et de bras articulés tels que 4.

La tête 1 est animée d'un mouvement vertical au-dessus d'un convoyeur d'acheminement 5, sous forme, par exemple, d'une succession de paniers à oeufs, tandis que ledit convoyeur est mû au-dessus d'une boîte à lumière 6, aménagée pour produire une lumière infrarouge pour les raisons évoquées ci-après.

La figure 1 montre en outre un convoyeur d'avacuation 7, sous forme d'un tapis à barreaux, qui passe au voisinage d'un récipient 8 de récupération. La tête 1 comporte une pluralité de ventouses, telles que 9, dont le nombre correspond à celui des logements d'oeufs de chaque panier du convoyeur 5, lesdits paniers étant par ailleurs ajourés par en-dessous pour laisser passer la lumière.

Une ventouse 9 est vue plus en détail à la figure 2. Il y a lieu de considérer l'appellation ventouse dans son sens le plus large. Il peut s'agir d'une sorte de cloche à vide en matière appropriée. Son rôle est de pouvoir s'appliquer de manière étanche sur un oeuf à tester, tel que 10. Cette application étanche a un double but; il s'agit, d'une part, d'éviter des entrées de lumière parasite au moment du mirage et, d'autre part, d'assurer la possibilité de préhension par aspiration, comme il sera expliqué ci-après. Chaque ventouse, en caoutchouc flexible par exemple, est pourvue, dans sa partie supérieure, d'un phototransistor à infrarouge 11, noyé dans une résine de polyester 12, et d'un conduit d'aspiration 13. Le phototransistor est connecté à un câble blindé 14.

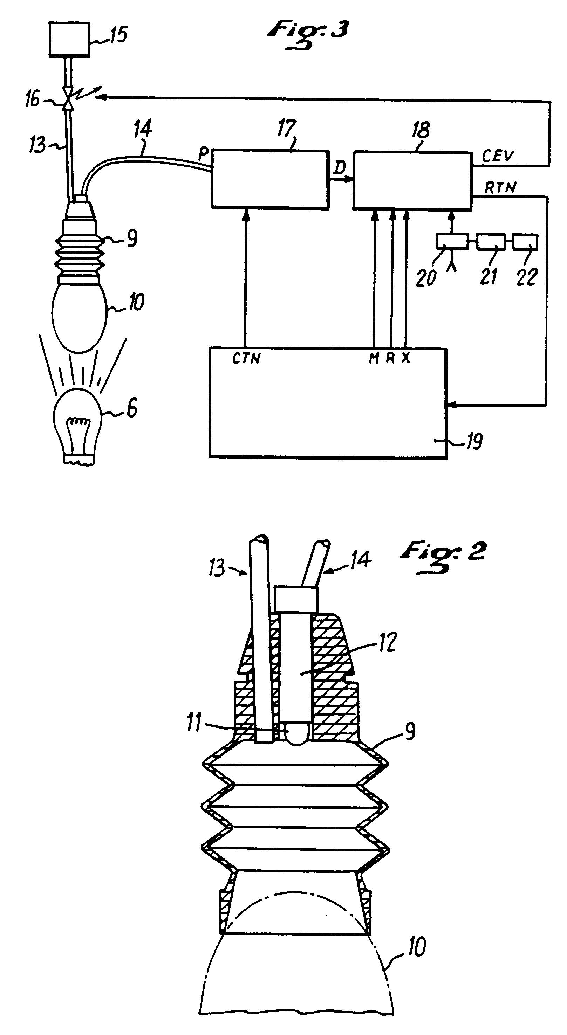

La figure 3 montre schématiquement le dispositif de la figure 2 relié à divers éléments.

Le conduit d'aspiration 13 est branché sur une pompe à vide 15, par l'intermédiaire d'une électrovanne 16, tandis que le câble 14 est connecté à une carte électronique 17, elle-même reliée à une autre carte 18. Les cartes 17 et 18 sont commandées par un automate de contrôle 19, destiné à générer plusieurs signaux respectivement CTN, M, R et X. En outre, la carte 18 recoit une alimentation électrique par l'intermédiaire d'un mesureur de courant 20, relié à un convertisseur analogiquedigital 21 et un calculateur 22 dont les fonctions seront précisées ci-après.

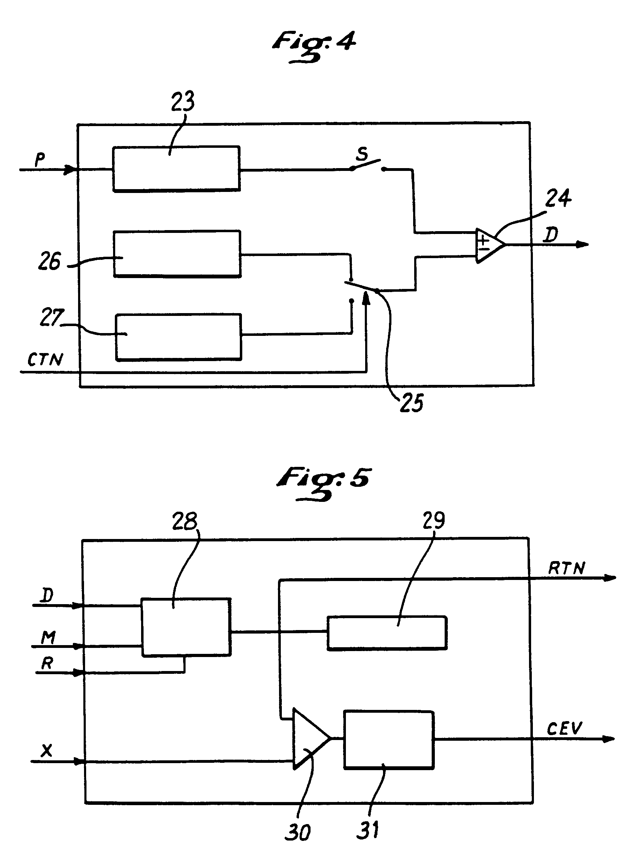

Le câble 14 connecté au phototransistor, achemine un signal P à l'entrée de la carte 17, comme le montrent les figures 3 et 4. Ce signal P est amplifié dans un étage d'amplification 23, qui est connecté par l'intermédiaire d'un interrupteur S à l'une des entrées d'un comparateur 24. L'autre entrée du comparateur 24 est connectée à un inverseur 25 qui est aménagé pour connecter au choix un appareil de seuil 26 ou un appareil de seuil 27 et qui est commandé par le signal CTN provenant de l'automate 19.

Le comparateur 24 délivre un signal de détection D qui est envoyé dans une mémoire 28 (figure 5) commandée par le signal M de mise en mémoire, provenant de l'automate 19 (figure 2), la mémoire 28 étant en outre susceptible de recevoir le signal R de vidage mémoire provenant de l'automate 19.

La sortie de la mémoire 28 est connectée à une diode de visualisation 29 et à l'une des entrées d'une porte électronique 30, du type "OU", dont l'autre entrée recoit le signal X provenant de l'automate 19 (figure 3). Le signal de sortie de la mémoire 28 est également bouclé en retour sur l'automate 19 (signal RTN des figures 3 et 5).

La sortie de la porte 30 est connectée à un relais 31 qui délivre un signal CEV qui, comme le montre la figure 3, est destiné à commander l'électrovanne 16.

Le fonctionnement de l'appareil est simple à comprendre et on va décrire ci-après, un cycle de fonctionnement.

L'appareil se trouve en position initiale, comme représenté à la figure 1, c'est-à-dire que la tête de mirage 1 est relevée. Le convoyeur 5 amène un plateau d'oeufs à tester sous la tête 1 qui amorce sa descente sur les oeufs, de telle sorte que chaque ventouse 9 s'applique sur un oeuf 10, comme le montre la figure 2.

Afin d'assurer une étanchéité parfaite de chaque ventouse, l'automate 19 permet d'envoyer le signal X, qui vient stimuler le relais 30 pour générer le signal de commande CEV, afin d'activer l'électrovanne 16 et de permettre, à l'aide du conduit 13 et de la pompe 15 de créer un vide partiel dans ladite ventouse. En même temps, le signal X ou un autre signal distinct (signal CTN par exemple), permet d'éteindre la boîte à lumière 6. Dans ces conditions, le phototransistor 11 de chaque ventouse ne devrait en principe délivrer aucun signal ou seulement des signaux P extrêmement faibles, même en sortie de l'étage d'amplification 23. Ce processus permet en fait de tester le bon état de chaque ventouse. Ce test, dit de "test noir" est réalisé au moyen de la commande CTN de l'automate 19 (commande "test noir") qui agit sur l'inverseur 25 (figure 4) pour connecter l'appareil 27 sur l'entrée du comparateur 24. L'appareil 27 est aménagé pour générer un signal qui correspond sensiblement à une absence de réception de lumière, c'est-à-dire à un seuil prédéterminé de contrôle (seuil test noir).

Dans ce cas, si la ventouse est correcte, le signal de détection D, provenant de la comparaison entre le signal P et le signal du seuil de contrôle, et qui arrive sur la mémoire 28, est pratiquement nul.

Par contre, le moindre défaut, entraîne le dépassement du seuil de contrôle, ce qui active la diode 29, visualisant ainsi la ventouse en défaut, tandis que le signal RTN de retour permet en revenant sur l'automate 19, par exemple, de stopper la machine.

Il est en outre intéressant de noter que l'interrupteur S, permet d'isoler (en l'ouvrant comme représenté à la figure 4), la ventouse défectueuse afin de pouvoir continuer le cycle de fonctionnement.

Après ce contrôle périodique ou systématique, on agit de nouveau sur l'inverseur 25 pour connecter l'appareil 26 (comme représenté sur la figure 4), lequel est aménagé pour créer un signal prédéterminé de seuil dit de mirage. Ce seuil dépend de l'état de transparence que l'on désire déceler. La boîte à lumière 6 est alors allumée, l'infrarouge utilisé permettant d'éviter les incidences extérieures et de procéder sans inconvénient dans un lieu éclairé naturellement ou artificiellement.

Le comparateur 24 va alors générer un signal D, soit très faible, soit nul, si le signal P, après amplification, dépasse le signal de seuil de mirage. Le signal M de mise en mémoire permet de mémoriser le signal D dans la mémoire 28. En cas de dépassement du seuil (oeuf considéré comme trop transparent), la diode 29 s'allume (visualisant la ventouse qui détecte un oeuf défectueux, et, surtout, le relais 31 reste stimulé par l'intermédiaire de la porte 30, même si le signal de commande X est annulé.

Après la mise à zéro du signal X, la tête 1 est remontée et il est clair que seuls les oeufs défectueux restent maintenus par aspiration (commande CEV active).

Il suffit alors de faire avancer le convoyeur 7, sous forme de tapis à barreaux, sous la tête 1, et de relâcher les oeufs relevés en supprimant le vide maintenu dans les ventouses. Pour annihiler l'aspiration, il suffit de couper l'alimentation électrique des électrovannes 16, par l'intermédiaire éventuel des relais 31. Pour supprimer l'aspiration, il est encore possible de simplement vider la mémoire 28 au moyen du signal de mise à zéro R (figure 3 et 5).

Après cette opération, le convoyeur 7 s'avance, comme le montre la figure 1 et déverse les oeufs défectueux dans le récipient de récupération 8.

Il est alors possible d'amener un nouveau plateau d'oeufs en avancant le convoyeur 5 et de récupérer les bons oeufs.

Il est possible aussi de transférer les bons oeufs du convoyeur 5 sur un autre convoyeur de transfert, schématisé en 32 sur la figure 1. Le passage du convoyeur 5 au convoyeur 32 est évidemment effectué avec la tête 1, qui vient prendre par aspiration les oeufs du convoyeur 5 pour les amener vers ledit convoyeur 32 (des moyens pour ce mouvement étant alors prévus sur la tête 1), à moins de prévoir, comme le montre la figure 1, un déplacement latéral possible du convoyeur 32, qui vient se placer sous la tête 1 pour recevoir les bons oeufs.

Comme le montre la figure 3, l'alimentation électrique des électrovannes peut être mesurée par un appareil de mesure 20 (mesure d'intensité et/ou de voltage et/ou de consommation), lequel est relié au convertisseur analogique-digital 21 qui délivre un signal au calculateur 22.

De la sorte, il est possible, en mesurant le courant électrique utilisé par les électrovannes au moment où la tête se relève avec les oeufs défectueux, de calculer par le calculateur 22, le nombre desdits oeufs défectueux. Ce type de calcul qui permet d'établir notamment des ratios entre les bons oeufs et les oeufs défectueux, peut bien sûr être effectué de toute autre manière, à partir du moment où l'appareil est capable de séparer et de visualiser les oeufs selon leur nature. La mesure du courant utilisé peut être prise sur une autre partie de l'appareil, de même qu'il est possible d'envoyer dans le calculateur, directement des signaux sous forme d'impulsions créées à partir de signaux de détection D au moment du mirage (c'est-à-dire de la comparaison entre lesdits signaux D et les signaux de seuil de mirage).

De très nombreuses modifications ou variantes peuvent évidemment être apportées sans sortir du cadre de l'invention. En dehors du nombre de ventouses 9, du nombre de convoyeurs et des mouvements entre eux qui peuvent différer par rapport à la description qui précède, d'autres particularités peuvent être imaginées. C'est ainsi, par exemple, que la lumière de type infrarouge n'est pas obligatoire, que les moyens de mise sous vide peuvent être réalisés sous forme de simples clapets, que les appareils 26 et 27 peuvent être plus nombreux et/ou réglables de manière, par exemple, à établir plusieurs seuils de test possible.

Les appareils 26 et 27 et l'inverseur 25 peuvent aussi être remplacés par un générateur unique capable de délivrer au choix plusieurs signaux, ...

## Revendications

1. Ovoscope trieur automatique, caractérisé en ce qu'il comporte une boîte à lumière (6) aménagée au-dessous d'une tête de mirage (1) qui est montée de manière mobile verticalement au-dessus des oeufs (10) à mirer et d'un convoyeur d'évacuation (7), et qui est munie d'au moins une ventouse (9) destinée à saisir par aspiration des oeufs à l'unité et équipée d'un moyen de mise sous vide et d'un moyen (11) sensible à la lumière, tandis qu'un dispositif de commande (31) du moyen de mise sous vide permet d'appliquer par aspiration chaque ventouse sur un oeuf (10) à mirer et d'en apprécier la transparence en fonction de l'importance de la lumière provenant de la boîte à lumière (6) et parvenant au moyen (11) sensible, tandis que le dispositif de commande est asservi au signal dudit moyen sensible à la lumière, de manière telle que ladite ventouse puisse retenir par aspiration l'oeuf lorsque le signal du moyen sensible à la lumière indique le dépassement d'une certaine transparence prédéterminée dudit oeuf et permettre de le déplacer verticalement en même temps que la tête de mirage pour le relâcher sur ledit convoyeur d'évacuation (7).

2. Ovoscope selon la revendication 1, caractérisé en ce que la tête de mirage (1) est montée de manière mobile verticalement également au-dessus d'un convoyeur d'acheminement (5) des oeufs à mirer, le tout de manière, après mirage, à évacuer les oeufs défectueux au moyen du convoyeur d'évacuation (7), à récupérer les bons oeufs au moyen du convoyeur d'acheminement (5) et à amener un nouveau lot d'oeufs à mirer au moyen dudit convoyeur d'acheminement.

3. Ovoscope selon la revendication 2, caractérisé en ce qu'il comporte en outre un convoyeur de transfert (32) destiné à recevoir, après mirage, les bons oeufs, qui sont transférés au moyen de la tête de mirage (1), du convoyeur d'acheminement (5), audit convoyeur de transfert (32).

4. Ovoscope selon l'une des revendications 1 à 3, caractérisé en ce que le moyen de mise sous vide de chaque ventouse (9) comporte un conduit d'aspiration (13) dont l'une des extrémités débouche dans le fond de ladite ventouse et dont l'autre extrémité est reliée à une pompe à vide (15) par l'intermédiaire d'une électrovanne (16).

5. Ovoscope selon l'une des revendications 1 à 4, caractérisé en ce que le moyen sensible à la lumière est un phototransistor (11) à infrarouge, tandis que la boîte à lumière (6) est aménagée pour produire de la lumière infrarouge.

6. Ovoscope selon l'une des revendications 1 à 5, caractérisé en ce qu'il comporte un système de contrôle qui permet de contrôler la détection correcte d'absence de lumière par chaque moyen sensible lorsque la boîte à lumière est éteinte et que la ventouse en cause est appliquée sous vide sur un oeuf.

7. Ovoscope selon la revendication 6, caractérisé en ce qu'il comporte un moyen de visualisation (29) du mauvais fonctionnement de la ventouse contrôlée, ainsi qu'un moyen (S) qui permet d'isoler ladite ventouse en cas de défaillance détectée.

8. Ovoscope selon l'une des revendications 1 à 7, caractérisé en ce qu'il comporte par ventouse (9), un comparateur (24) aménagé pour comparer le signal (P) du moyen correspondant sensible à la lumière (11) avec au moins un signal prédéterminé, généré par un générateur de signaux de seuil (26,27), de manière à créer un signal de détection (D) qui est fonction de la comparaison desdits signaux.

9. Ovoscope selon la revendication 8, caractérisé en ce que le générateur (26) de signaux de seuil est aménagé pour générer au moins un signal prédéterminé de comparaison qui correspond à un seuil choisi de transparence, de manière à pouvoir créer un signal de détection (D) qui est fonction du dépassement ou non de ce seuil.

10. Ovoscope selon l'une des revendications 6 et 7 et l'ensemble des revendications 8 et 9, caractérisé en ce que le générateur (27) de signaux de seuil est aménagé pour générer en outre un signal prédéterminé de comparaison qui correspond à un seuil d'absence de lumière, de manière à pouvoir créer un signal de détection, dit de "test noir", qui est fonction du dépassement ou non de ce seuil.

11. Ovoscope selon l'une des revendications 9 et 10, caractérisé en ce que le signal de détection (D), après mémorisation, sert à agir sur la commande (31) du moyen de mise sous vide, de manière à maintenir la mise sous vide de la ventouse correspondante lorsque ledit signal indique un dépassement du seuil choisi.

12. Ovoscope selon l'ensemble des revendications 8 à 11, caractérisé en ce qu'il comporte un automate de contrôle (19), qui permet de commander le choix du signal prédéterminé de comparaison, de commander la mise en mémoire du signal de détection et de commander un éventuel arrêt de l'appareil lorsque le signal de test noir branché en retour (RTN) sur ledit automate (19), est mauvais.

13. Ovoscope selon l'une des revendications 1 à 12, caractérisé en ce qu'il comporte un moyen de mesure du courant électrique utilisé par une partie au moins de l'appareil et un calculateur aménagé de manière à déterminer en fonction des informations dudit appareil de mesure, des rapports numériques entre le nombre d'oeufs défectueux et le nombre des bons oeufs.

## Claims

1. Automatic sorting ovoscope, characterised in that it comprises a light box (6) arranged under a candling head (1) which is mounted so as to be moveable vertically over the eggs (10) to be candled and a discharge converter (7), and which is fitted with at least one suction cup (9) designed to seize eggs one at a time by suction and equipped with an evacuating means and a light-sensitive means (11), while a device (31) controlling the evacuating means makes it possible to apply each suction cup by suction to an egg (10) to be candled and to determine its transparency according to the amount of light coming from the light box (6) and reaching the light-sensitive means (11), while the control device is controlled by the signal from said light-sensitive means, so that said suction cup can hold onto the egg by suction when the signal from the light-sensitive means indicates that the transparency of said egg exceeds a certain pre-determined level and allow it to be moved vertically at the same time as the candling head in order to release it onto said discharge conveyor (7).

2. Ovoscope according to claim 1, characterised in that the candling head (1) is mounted so as to be additionally moveable vertically over a feeding conveyor (5) for the eggs to be candled, the lot so that, after candling, the bad eggs are discharged by means of the discharge conveyor (7), the good eggs are recovered by means of the feeding conveyor (5) and a new batch of eggs is brought up for candling by means of said feeding conveyor.

3. Ovoscope according to claim 2, characterised in that it additionally comprises a transfer conveyor (32) designed to receive the good eggs after candling, these being transferred from the feeding conveyor (5) to said transfer conveyor (32) by means of the candling head (1).

4. Ovoscope according to one of claims 1 to 3, characterised in that the means for evacuating each suction cup (9) comprises a suction pipe (13) one of the ends of which debouches into the bottom of said suction cup and the other end of which is connected to a vacuum pump (15) through the intermediary of an electro-magnetic valve (16).

5. Ovoscope according of one of claims 1 to 4, characterised in that the light-sensitive means is an infra-red phototransistor (11), while the light box (6) is arranged to produce infra-red light.

6. Ovoscope according to one of claims 1 to 5, characterised in that it comprises a monitoring system which makes it possible to monitor the correct detection of absence of light by each light-sensitive means when the light box is extinguished and the suction cup in question is applied by vacuum to an egg.

7. Ovoscope according to claim 6, characterised in that it comprises a means (29) for visually displaying the incorrect operation of the suction cup being monitored, and a means (S) which makes it possible to isolate said suction cup in the event of a fault being detected.

8. Ovoscope according to one of claims 1 to 7, characterised in that for each suction cup (9) it comprises a comparator (24) arranged to compare the signal (P) from the corresponding light-sensitive means (11) with at least one pre-determined signal generated by a threshold signal generator (26, 27), so as to create a detection signal (D) which depends on the comparison of said signals.

9. Ovoscope according to claim 8, characterised in that the threshold signal generator (26) is arranged to generate at least one pre-determined comparison signal which corresponds to a selected transparency threshold, so as to be able to create a detection signal (D) which depends on whether this threshold is exceeded or not.

10. Ovoscope according to one of claims 6 and 7 and all of claims 8 and 9, characterised in that the threshold signal generator (27) is arranged to additionally generate a predetermined comparison signal which corresponds to a no-light threshold, so as to be able to create a so-called "dark test" detection signal which depends on whether this threshold is exceeded or not.

11. Ovoscope according to one of claims 9 and 10, characterised in that after storage the detection signal (D) is used to act on the device (31) controlling the evacuating means so as to maintain the evacuation of the corresponding suction cup when said signal indicates that the selected threshold is exceeded.

12. Ovoscope according to all of claims 8 to 11, characterised in that it comprises an automatic controller (19) which makes it possible to initiate the selection of the pre-determined comparison signal, to initiate the storage of the detection signal and to initiate a stoppage of the device when the dark test signal (RTN) returned to said automatic controller (19) is incorrect.

13. Ovoscope according to one of claims 1 to 12, characterised in that it comprises a means for measuring the electrical current used by at least one part of the device and a processor arranged so as to determine numerical ratios between the number of bad eggs and the number of good eggs according to the information from said measuring device.

## Patentansprüche

1. Automatisches Eierprüf- und sortiergerät dadurch gekennzeichnet, daß es einen Leuchtkasten (6) aufweist, der unterhalb eines Prüfkopfes (1) angeordnet ist, welcher in senkrechter Richtung bewegbar oberhalb der zu prüfenden Eier (10) und eines Abführungsförderers (7) montiert ist und der mit mindestens einem Saugnapf (9) versehen ist, der dazu bestimmt ist, Eier als ganze Einheit durch Ansaugen zu ergreifen, und mit einem Mittel zum Untervakuumsetzen sowie einem lichtempfindlichen Mittel (11) ausgerüstet ist, während es eine Vorrichtung (31) zum Steuern des Mittels zum Untervakuumsetzen ermöglicht, jeden Saugnapf durch Ansaugen auf einem zu prüfenden Ei (10) zu befestigen und dessen Transparenz in Abhängigkeit von der vom Leuchtkasten (6) ausgehenden und zum lichtempfindlichen Mittel (11) gelangenden Lichtmenge zu beurteilen, wohingegen die Steuervorrichtung vom Signal des besagten lichtempfindlichen Mittels derart angesteuert wird, daß der besagte Saugnapf, wenn das Signal des lichtempfindlichen Mittels das Überschreiten einer bestimmten vorgegebenen Transparenz des besagten Eies anzeigt, das Ei durch Ansaugen festhalten und es gleichzeitig mit dem Prüfkopf in vertikaler Richtung versetzen kann, um es auf dem besagten Abführungsförderer (7) loszulassen.

2. Eierprüfgerät nach Anspruch 1 dadurch gekennzeichnet, daß der Prüfkopf (1) in senkrechter Richtung bewegbar außerdem oberhalb eines Zuführungsförderers (5) für die zu prüfenden Eier montiert ist, dies alles in der Weise, daß nach der Prüfung die mangelhaften Eier mittels des Abführungsförderers (7) abgeführt, die guten Eier mittels des Zuführungsförderers (5) zurückgeholt und ein neuer Satz zu prüfender Eier mittels des besagten Zuführungsförderers zugeführt wird.

3. Eierprüfgerät nach Anspruch 2 dadurch gekennzeichnet, daß es außerdem einen Weiterführungsförderer (32) aufweist, der dazu bestimmt ist, nach der Prüfung die guten Eier aufzunehmen, die mittels des Prüfkopfes (1) vom Zuführungsförderer (5) auf den besagten Weiterführungsförderer (32) überführt worden sind.

4. Eierprüfgerät nach einem der Ansprüche 1 bis 3 dadurch gekennzeichnet, daß das Mittel zum Untervakuumsetzen jedes Saugnapfes (9) eine Saugleitung (13) aufweist, deren eines Ende in den Boden des besagten Saugnapfes einmündet und deren anderes Ende mit einer Vakuumpumpe (15) unter Zwischenschaltung eines Magnetventils (16) verbunden ist.

5. Eierprüfgerät nach einem der Ansprüche 1 bis 4 dadurch gekennzeichnet, daß das lichtempfindliche Mittel ein Phototransistor (11) für Infrarotlicht ist, während der Leuchtkasten (6) für die Erzeugung von Infrarotlicht ausgerüstet ist.

6. Eierprüfgerät nach einem der Ansprüche 1 bis 5 dadurch gekennzeichnet, daß es ein Prüfsystem aufweist, welches es ermöglicht, die korrekte Erfassung der Abwesenheit von Licht an jedem lichtempfindlichen Mittel zu überprüfen, wenn der Leuchtkasten ausgeschaltet und der in Frage kommende Saugnapf unter Vakuum auf ein Ei aufgesetzt ist.

7. Eierprüfgerät nach Anspruch 6 dadurch gekennzeichnet, daß es ein Mittel (29) zur optischen Anzeige des schlechten Funktionierens des geprüften Saugnapfes aufweist, sowie ein Mittel (S), welches es ermöglicht, den besagten Saugnapf bei einem erfaßten Ausfall abzutrennen.

8. Eierprüfgerät nach einem der Ansprüche 1 bis 7 dadurch gekennzeichnet, daß es pro Saugnapf (9) einen Vergleicher (24) aufweist, der für den Vergleich des Signals (P) des entsprechenden lichtempfindlichen Mittels (11) mit mindestens einem vorgegebenen Signal ausgerüstet ist, welches von einem Schwellensignalgenerator (26, 27) erzeugt wird, derart, daß ein Erfassungssignal (D) erzeugt wird, das vom Vergleich der besagten Signale abhängig ist.

9. Eierprüfgerät nach Anspruch 8 dadurch gekennzeichnet, daß der Schwellensignalgenerator (26) für die Erzeugung mindestens eines vorgegebenen Vergleichssignals ausgerüstet ist, welches einem gewählten Schwellenwert für die Transparenz entspricht derart, daß ein Erfassungssignal (D) erzeugt werden kann, das vom Überschreiten oder Nichtüberschreiten dieses Schwellenwertes abhängig ist.

10. Eierprüfgerät nach einem der Ansprüche 6 und 7 und der Gesamtheit der Ansprüche 8 und 9 dadurch gekennzeichnet, daß der Schwellensignalgenerator (27) außerdem für die Erzeugung eines vorgegebenen Vergleichssignal ausgerüstet ist, welches einem Schwellenwert für die Abwesenheit von Licht entspricht derart, daß ein "Schwarztest" genanntes Erfassungssignal erzeugt werden kann, das vom Überschreiten oder Nichtüberschreiten dieses Schwellenwertes abhängig ist.

11. Eierprüfgerät nach einem der Ansprüche 9 und 10 dadurch gekennzeichnet, daß das Erfassungssignal (D) nach seiner Einspeicherung dazu dient, auf die Steuerung (31) des Mittels zum Untervakuumsetzen einzuwirken derart, daß das Untervakuumsetzen des entsprechenden Saugnapfes aufrechterhalten wird, wenn das besagte Signal eine Überschreitung des gewählten Schwellenwertes anzeigt.

12. Eierprüfgerät nach der Gesamtheit der Ansprüche 8 bis 11 dadurch gekennzeichnet, daß es einen Prüfautomaten (19) aufweist, der es ermöglicht, die Wahl des vorgegebenen Vergleichssignals zu steuern, und das Einspeichern des Erfassungssignals und ein eventuelles Anhalten des Gerätes zu steuern, wenn das abgezweigte und zu dem besagten Automaten (19) zurückgeführte Signal (RTN) des Schwarztestes schlecht ist.

13. Eierprüfgerät nach einem der Ansprüche 1 bis 12 dadurch gekennzeichnet, daß es ein Meßmittel für den von mindestens einem Teil des Gerätes verbrauchten elektrischen Strom aufweist und einen Rechner, welcher derart ausgerüstet ist, daß er in Abhängigkeit von Informationen des besagten Meßgerätes die Zahlenverhältnisse zwischen der Anzahl der mangelhaften Eier und der Anzahl der guten Eier bestimmt.
